# FASCICULE DE BREVET EUROPEEN

(11) **EP 0 739 870 B1**
(45) Date de publication et mention de la délivrance du brevet: **04.09.2002**
(21) Numéro de dépôt: 96400856.9
(22) Date de dépôt: 23.04.1996
(51) Int. Cl.: C07B 43/08, C07C 253/30, C07C 319/20, C07C 323/60

(54) **Procédé de condensation de l'acide cyanhydrique avec un aldéhyde**
Verfahren zur Kondensation von Cyanwasserstoff mit einem Aldehyd
Process for the condensation of hydrogen cyanide with an aldehyde

(30) Priorité: 24.04.1995 FR 9504865
(43) Date de publication de la demande: 30.10.1996
(73) Titulaire: AVENTIS ANIMAL NUTRITION S.A., 92160 Antony (FR)
(72) Inventeur: Casse, Claude, 69150 Decines Charpieu (FR); Kress, Frédéric, 38200 Vienne (FR); Morel, Philippe, 38200 Chuzelles (FR)
(74) Mandataire: Mérigeault, Shona

(56) Documents cités:
- EP-A- 0 330 527
- DE-A- 4 235 295
- US-A- 2 745 745
- SYNTHESIS, no. 7, Juillet 1990, STUTTGART, DE, pages 575-578, XP002009640 T. ZIEGLER, ET AL.: "Ein einfacher Zugang zu (R)-alpha-Hydroxycarbonsäuren und (R)-1-Amino-2-alkoholen aus (R)-Cyanhydrinen"
- JOURNAL OF THE AMERICAN CHEMICAL SOCIETY, vol. 113, no. 18, 28 Août 1991, WASHINGTON, DC, US, pages 6992-6996, XP002009641 V.I. OGNYANOV, ET AL.: "Preparation of chiral cyanohydrins by an oxynitrilase- mediated transacylation"
- JOURNAL OF THE AMERICAN CHEMICAL SOCIETY, vol. 70, no. 4, 3 Mai 1948, WASHINGTON, DC, US, pages 1450-1451, XP002009642 E. PIERSON, ET AL.: "Synthesis of DL-methionine"
- TETRAHEDRON: ASYMMETRY, vol. 4, no. 5, Mai 1993, OXFORD, GB, pages 893-902, XP000162125 Y. LU, ET AL.: "A simple total synthesis of naturally ocurring hydroxy-amino acids by enzymatic kinetic resolution"
- SYNTHETIC COMMUNICATIONS, vol. 21, no. 12-13, 1991, NEW YORK, US, pages 1387-1391, XP002009644 P. ZANDBERGEN, ET AL.: "Synthesis of optically active cyanohydrins using almond meal"
- TETRAHEDRON: ASYMMETRY, vol. 5, no. 8, Août 1994, OXFORD GB, pages 1567-1578, XP002009645 I. TELLITU, ET AL.: "A simple enzymatic synthesis of (3S,4R)-(+)-4-hydroxy- 3-phenyltetrahydroisoquinolines"
- TETRAHEDRON, vol. 46, no. 3, 1990, OXFORD, GB, pages 979-986, XP002009646 J. BRUSSE, ET AL.: "Synthesis of optically active silyl protected cyanohydrins"

## Description

La présente invention concerne de nouveaux catalyseurs de condensation de l'acide cyanhydrique sur un aldéhyde. Elle concerne également le procédé de condensation de l'acide cyanhydrique sur un aldéhyde.

Il est connu depuis longtemps, par exemple selon le brevet US-A-4 518 801 de préparer la méthionine par hydrolyse de la 5-(béta méthyl mercaptoéthyl)-hydantoïne en présence de 0,3 à 5 équivalents d'hydroxyde alcalin, de carbonate alcalin ou de leur mélange puis de séparer le méthioninate alcalin obtenu des sels coproduits durant l'hydrolyse et enfin d'hydrolyser le sel alcalin obtenu en méthionine. L'hydantoïne étant préparée par contact entre la cyanhydrine de l'aldéhyde méthylthiopropionique, l'ammoniac et du dioxyde de carbone.

II est également connu exemple selon le brevet US-A 4 960 932 de préparer la méthionine par un procédé comprenant quatre étapes. Dans la première étape on prépare le méthylthio-α-hydroxy butyronitrile par condensation de l'aldéhyde méthylthiopropionique et de l'acide cyanhydrique en présence de triéthylamine. Ensuite, dans une deuxième étape on met en contact le méthylthio-α-hydroxybutyronitrile avec l'ammoniac pour former le méthylthio α aminobutyronitrile qui est ensuite hydrolysé dans une troisième étape en présence d'une cétone et d'un hydroxyde alcalin pour former le méthylthiobutyramide qui est finalement hydrolysé en méthioninate alcalin puis saponifié par un acide.

Dans le cas de la préparation de l'acide méthylthio-α-hydroxy butyrique, encore appelé hydroxyanalogue de la méthionine, le méthylthio-α -hydroxybutyronitrile obtenu par réaction de l'aldéhyde méthylthio propionique avec l'acide cyanhydrique, dans un milieu contenant de la pyridine ou une amine (voir brevet US-A-2 745 745 colonne 2 lignes 52 à 55) est hydrolysé par l'acide sulfurique pour former intermédiairement l'amide de l'acide méthylthio -α-hydroxy butyrique et finalement l'acide méthylthio α hydroxy butyrique (voir le brevet européen EP-A-330 527 ou le brevet US-A-2 745 745).

Les bases utilisées lors de la mise en contact de l'aldéhyde méthylthio propionique avec l'acide cyanhydrique augmentent la vitesse de réaction, mais entraînent rapidement une dégradation de la cyanhydrine formée et une dégradation de l'aldéhyde de départ avec formation d'une solution fortement colorée. Ces bases sont, en plus, volatiles et toxiques ce qui est un lourd handicap lors d'une exploitation industrielle car elles exigent lors de leur utilisation des conditions de sécurité et de recyclage qui dans tous les cas sont couteuses. En plus de ces inconvénients ces bases étant volatiles peuvent provoquer des accumulations locales rendant le milieu par endroit très basique ce qui provoque une augmentation de coloration, d'une part par polymérisation de l'acide cyanhydrique et d'autre part par dégradation de l'aldéhyde méthylpropionique.

Il est apparu de façon tout à fait étonnante que les réactions de dégradation pouvaient être réduites et la réaction de condensation favorisée si on opérait dans une zone de pH bien délimitée et en l'absence des amines toujours utilisées dans l'art antérieur.

Dans le document "Tetrahehydron: Asymmetry (vol. 4, No. 5, May 1993, pages 893-902, Y.Lu, et Al.)"est divulgué un procédé de condensation d'un aldéhyle formé in situ avec l'acide cyanhydrique formé in situ par un tampon permettant de fixer le pH au-dessus de 4, le tampon utilisé ne contenant pas d'aminé.

Enfin DE-A-4235295 divulgue un procédé de condensation d'acide cyanhydrique et l'aldéhyde, l'aldéhyde méthylthiopropionique, sans tampon en présence d'aminé ou ou de pyridine.

La présente invention concerne donc la condensation d'un aldéhyde avec l'acide cyanhydrique en présence d'un tampon permettant de fixer le pH de la solution au dessus de 4,0 à l'exclusion des amine. Ce procédé est caractérisé en ce que l'aldéhyde est un aldéhyde aliphatique contenant 1 à 6 atomes de carbone éventuellement substitué par un groupe alkyle, alkoxy ou alkylthio.

Le pH de la solution est de préférence fixé entre 4,0 et 6,0. Le pH de la solution est encore plus préférentiellement fixé à 5. On préfère selon une meilleure manière de mettre en oeuvre l'invention, pour permettre notamment la catalyse d'une réaction par absorption réactive c'est à dire par contact entre un gaz tel que l'acide cyanhydrique et un liquide tel que le méthylthiopropionaldéhyde, un tampon qui est de préférence non volatil et non toxique, par opposition aux amines volatiles utilisées dans l'art antérieur.

Parmi les tampons permettant de fixer le pH dans la zone considérée il est préférable d'utiliser un tampon choisi parmi les mélanges suivants qui sont cités à titre d'exemples et ne sont pas limitatifs de l'invention:
- acide citrique et citrate de sodium ou acide citrique et hydroxyde de sodium
- acide phosphorique et phosphate acide de sodium ou acide phosphorique et hydroxyde de sodium,
- acide succinique et de succinate de sodium ou acide succinique et hydroxyde de sodium,
- acide acétique et acétate de sodium ou acide acétique et hydroxyde de sodium,
- hydrogénophtalate de potassium et hydroxyde de sodium.

D'une manière plus générale on peut utiliser comme tampon les mélanges de sels alcalins d'acide et d'acide ou les mélanges d'acide et d'hydroxyde alcalin.

L'utilisation d'un tampon présente l'avantage d'une part d'éviter la dégradation de la matière première et du produit désiré et d'autre part de neutraliser les acides de stabilisation de l'acide cyanhydrique tels que l'acide sulfurique.

Parmi les adéhydes on préfère utiliser les aldéhydes aliphatiques contenant 1 à 6 atomes de carbone éventuellement substitués par un groupe alkyle ou alkoxy ou alkylthio, on préfère tout particulièrement utiliser l'aldéhyde méthylthio propionique.

Selon une meilleure façon de mettre en oeuvre l'invention, on prépare le tampon in situ par neutralisation de l'acide citrique par la soude selon un rapport molaire acide citrique/soude compris entre 0.3 et 0.7 et de. préférence d'environ 0.5.

Selon une meilleure manière de mettre en oeuvre l'invention, on utilise selon une première technologie, pour la condensation : un mélange gazeux d'acide cyanhydrique contenant en volume environ 7% d'acide cyanhydrique et une solution aqueuse d'aldéhyde. L'acide cyanhydrique est préparé notamment selon le procédé Andrussow puis il est débarrassé de l'ammoniac qu'il contient.

En particulier l'invention concerne un procédé de condensation entre un aldéhyde et l'acide cyanhydrique en présence d'un tampon permettant de fixer le pH de la solution au dessus de 4 à l'exclusion des amines, ledit aldéhyde étant un aldéhyde contenant 1 à 6 atomes de carbone, éventuellement substitué par un groupe alkyle, alkoxy ou alkylthio, caractérisé en ce que la condensation est une réaction entre l'aldéhyde liquide et une solution aqueuse d'acide cyanhydrique.

Ainsi selon une deuxième manière de mettre en oeuvre l'invention, une solution aqueuse d'acide cyanhydrique dans l'eau contenant environ 20% en poids d'acide cyanhydrique, est mise en contact avec l'aldéhyde. Dans tous les cas le rapport molaire entre l'acide cyanhydrique et l'aldéhyde est de préférence légèrement excédentaire en acide cyanhydrique. On préfère, ainsi, utiliser un excès molaire compris entre 0,1 et 10% et encore plus préférentiellement compris entre 2 et 5%.

Le tampon utilisé comme catalyseur de condensation est utilisé de façon à fixer le pH dans la zone déterminée il est de préférence utilisé par exemple pour le tampon citrique-citrate selon un rapport molaire par rapport à l'aldéhyde compris entre 0,01 et 0,0001 et de préférence d'environ 0,001. Il est utilisé en solution aqueuse de telle façon que la cyanhydrine formée se présente, à la sortie de la colonne, selon, notamment, une concentration pondérale d'environ 50%.

La réaction peut s'effectuer comme indiqué précédemment entre un gaz et un liquide, elle s'effectue alors de préférence dans un contacteur gaz-liquide tel que:
- une colonne à plateaux
- une colonne à garnissage
- une colonne à bulles
- un réacteur tubulaire
- une colonne à gouttes
- un réacteur à cuve agitée mécaniquement
- un réacteur à jet immergé

Selon le premier dispositif de mise en oeuvre qui est préféré, c'est à dire lorsqu'on utilise une colonne d'absorption réactive à plateaux, le flux gazeux contenant l'acide cyanhydrique est introduit en bas de la colonne à plateaux et la solution aqueuse tamponnée d'aldéhyde est introduite en tête de colonne. A la place de la solution aqueuse d'aldéhyde il est possible d'introduire en tête de colonne des arrivées de flux indépendantes, c'est à dire une arrivée d'eau, une arrivée de solution tamponnée aqueuse et une arrivée de solution d'aldéhyde. Il est évident que l'homme de l'art adaptera le nombre de flux au dispositif qu'il aura l'intention d'utiliser.

Avantageusement en tête de la colonne d'absorption réactive on installe une colonne supplémentaire de lavage pour récupérer à partir des gaz issus de la colonne d'absorption réactive, l'aldéhyde subsistant ainsi que les traces d'acide cyanhydrique n'ayant pas réagi. Cette colonne de lavage permet un échange entre les gaz introduits en pied et de l'eau introduite en tête. On peut utiliser, par exemple, pour ce lavage des colonnes à plateaux ou à garnissage. L'eau récupérée en bas de cette colonne est avantageusement réintroduite dans la colonne d'absorption réactive et les gaz épurés sont envoyés à l'incinérateur.

Si la réaction est effectuée entre une solution aqueuse d'acide cyanhydrique et l'aldéhyde elle est de préférence réalisée en réacteur tubulaire tel qu'un réacteur piston adiabatique. Dans tous les cas la réaction est rapide et exothermique.

Selon le premier mode de mise en oeuvre de l'invention, la réaction s'effectue de préférence à une température comprise entre 50 et 100°C et de préférence à environ 70°C et à la pression atmosphérique quand on opère en colonne ouverte. La température est régulée par la quantité d'eau introduite avec le tampon.

Lorsque l'on opère en réacteur tubulaire, selon le deuxième mode de mise en oeuvre de l'invention, la température est de préférence fixée entre 30 et 110°C et la pression est notamment comprise entre 1 et 10 bars

La cyanhydrine de l'aldéhyde de départ, obtenue par l'une quelconque des méthodes de mise en oeuvre de l'invention et notamment la cyanhydrine de l'aldéhyde méthylthiopropionique est soit aminée, soit traitée par un mélange d'ammoniac et de dioxyde de carbone pour conduire ensuite à la méthionine soit directement hydrolysée en acide méthylthio-α-hydroxy butyrique.

La présente invention sera plus complètement décrite à l'aide des exemples suivants.

### EXEMPLE 1

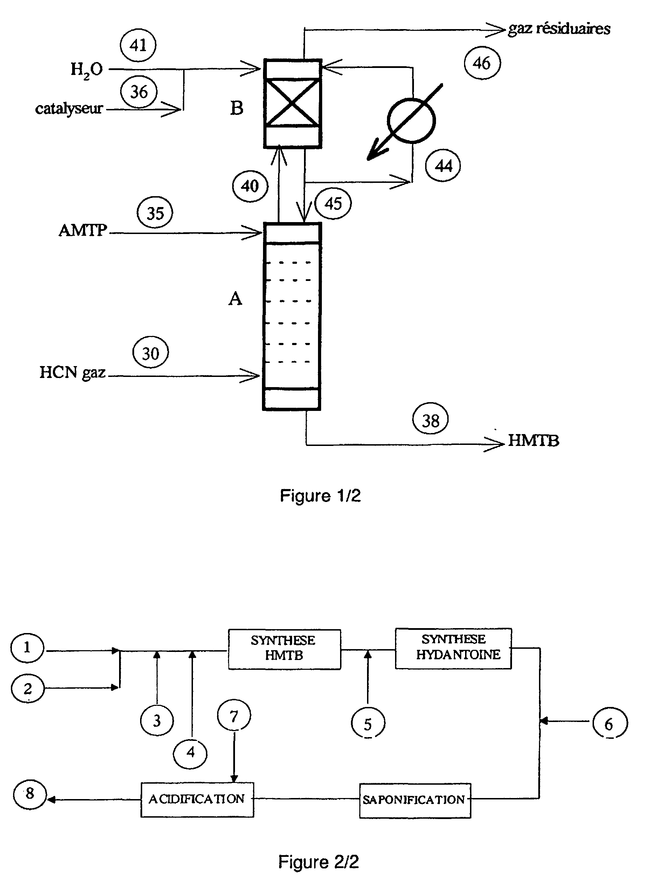

On utilise une colonne A, OLDERSHAW™ de 50mm de diamètre équipé de 20 plateaux à trous et d'une hauteur de 1 mètre et une colonne B, garnie, du même diamètre d'une hauteur de 20 cm équipée d'un garnissage multiknit. Le schéma de ce dispositif est annexé (voir figure 1/2).

On introduit dans la colonne A, par l'alimentation 30, 1556,2 g/h d'un gaz brut de synthèse d'acide cyanhydrique contenant en poids :
- HCN 109,12
- CH₄ 0,77
- CO 88,45
- CO₂ 33,02
- H₂ 18,12
- N₂ 895,46
- H₂O 402,75
- divers 8,52

On introduit dans la colonne A, par l'alimentation 35, de l'aldéhyde méthylthiopropionique avec un débit de 408,36 g/h, par l'alimentation 41 en tête de la colonne B on introduit 150 g/h d'eau et par l'alimentation 36 4,89 g/h d'un mélange de 1,13 g d'acide citrique et 0,49 g de soude.

Le pH de la colonne est de 5.

On recueille en bas de la colonne A, 1013 g/h d'une solution aqueuse contenant:
- 2-hydroxy méthylthiobutyronitrile 514,25
- HCN 1,54
- aldéhyde méthylthiopropionique 0,04
- H₂O 495,85
- acide citrique 1,13
- soude 0,49

Les gaz issus de la colonne A sont envoyés dans la colonne B à un débit de 1531 g/h et s'échappe de la colonne B à un débit de 1106 g/h ils sont constitués de :
- HCN 1,65
- CH₄ 0,77
- CO 88,45
- CO₂ 33,02
- H₂ 18,12
- N₂ 895,46
- H₂O 60,68
- divers 8,52

Ces gaz sont envoyés à l'incinérateur. Dans la colonne B, on impose un reflux (flux 44) vers la base de ladite colonne de 11181,91 g/h d'une solution aqueuse refroidie contenant :
- HCN 3,90
- N₂ 7,72
- H₂O 10434,91
- aldéhyde méthylthiopropionique 0,09
- hydroxy méthylthiobutyronitrile 703,59
- acide citrique 22,12
- soude 9,59

En sortie (flux 45) de la colonne B sont renvoyés vers la colonne A à un débit de 580,06 g/h une solution aqueuse ayant la même composition que la solution de reflux de la colonne B.

### EXEMPLE 2 Ajustement du rapport molaire NaOH/acide citrique

On reproduit l'exemple 1 en modifiant la valeur du pH. En fonction du pH on mesure, sur différents plateaux de la colonne, le taux de transformation de l'aldéhyde méthylthio propionique

| | pH 5,6 | pH 4,7 | pH 3,6 | pH 4,5 | pH 5,2 |
|---|---|---|---|---|---|
| 2^{ème} plateau | 50,32 | 38,31 | 39,00 | 47,29 | 73,92 |
| 4^{ème} plateau | 95,74 | 53,48 | 47,17 | 49,82 | 82,21 |
| 7^{ème} plateau | 100,00 | 96,15 | 70,09 | 69,86 | 98,79 |
| 9^{ème} plateau | 100,00 | 99,86 | 75,76 | 73,92 | 99,95 |
| 11^{ème} plateau | 100,00 | 99,94 | 84,21 | 84,17 | 99,99 |
| 13^{ème} plateau | 100,00 | 99,96 | 90,84 | 99,63 | 100,00 |
| 15^{ème} plateau | 100,00 | 99,98 | 98,48 | 99,93 | 100,00 |
| 17^{ème} plateau | 100,00 | 100,00 | 98,30 | 99,95 | 100,00 |
| 19^{ème} plateau | 100,00 | 100,00 | 99,27 | 99,87 | 100,00 |

L'exemple à pH 3,6 ne fait pas partie de l'invention.

Les solutions d'hydroxy méthylthio butyronitrile obtenues en fin de réaction sont introduites dans une étuve maintenue à 70°C pour étudier la dégradation dudit nitrile en fonction du temps. La dégradation est estimée par mesure de la coloration APHA selon la norme internationale ISO 2211-1973.

| pH tampon | pH 5,6 | pH 4,7 | pH 3,6 | pH 4,5 | pH 5,2 |
|---|---|---|---|---|---|
| pH du nitrile | 7,2 | 5,2 | 3,8 | 4,7 | 6,6 |
| durée d'étuvage (h) | 4 | 48 | 48 | 48/72 | 72 |
| coloration APHA avant étuvage | 966 | 190 | 300 | 238 | 881 |
| coloration APHA après étuvage | 17380 | 1071 | 1642 | 333/2837 | 8769 |

L'exemple à pH 3,6 ne fait pas partie de l'invention.

### EXEMPLE 3

### Synthèse de l'hydroxy-2 méthylthio butyronitrile

On charge sous agitation dans un réacteur en verre avec une double enveloppe de 150 ml:

| | |
|---|---|
| aldéhyde méthylthio propionique | 47,43 g |
| eau | 30,97 g |

Le milieu est hétérogène (2 phases liquides non miscibles). On ajoute 0,6669 g de solution catalytique. La solution catalytique est obtenue par mélange de :

| | |
|---|---|
| NaOH | 9g |
| acide citrique | 20,77g |
| eau | 70,30g |

La température du milieu s'établit aux alentours de 20°C. Le pH imposé par le système catalytique est voisin de 5.

Par l'intermédiaire d'une ampoule de coulée on introduit le plus rapidement possible 42,37 g d'une solution aqueuse d'acide cyanhydrique à 30,70% en masse. Instantanément la température du milieu réactionnnel s'élève jusqu'à 70°C. Ceci correspond à la chaleur de réaction. Le niveau de température (70°C) est maintenu dans la masse réactionnelle pendant une durée de 5mn par circulation d'un fluide chaud dans la double enveloppe.

Pendant la durée de la réaction le pH reste aux alentours de 5. A l'issue de ce temps de séjour à 70°C on a vérifié que le rendement de la réaction de synthèse du méthylthiobutyronitrile par rapport à l'aldéhyde méthylthiopropionique est de 100%.

Le milieu de réaction est devenu homogène dès que la température et le niveau de conversion de l'aldéhyde méthylthiopropionique ont atteint un niveau suffisant.

### EXEMPLE 4 deuxième méthode de mise en oeuvre de l'invention

On condense en continu dans un réacteur adiabatique l'aldéhyde méthylthiopropionique avec une solution aqueuse d'acide cyanhydrique à 20%. Cette solution est préparée à partir d'acide cyanhydrique liquide à 100% et d'eau. Le catalyseur de la réaction est ajouté en ligne dans l'eau. Selon le schéma de la figure 2/2, on introduit l'eau à un débit de 1941 kg/h ① le catalyseur acide citrique/citrate de sodium à un débit de 10Kg/h ②, la solution d'acide cyanhydrique à 20% à un débit de 398 kg/h ③ et l'aldéhyde méthylthio propionique ④ à un débit de 1444 kg/h.

La solution catalytique ② est préparée par addition de 390 g de soude aqueuse à 50% et 450 g d'acide citrique anhydre dans 1340 g d'eau. Le pH de la solution est ajusté à 5,0 ± 0,2 par ajout de l'un ou l'autre des deux réactifs.

L'hydroxyméthylthiobutyronitrile formé réagit avec les eaux ammoniacales ⑤ qui sont introduites à un débit de 8400 kg/h. Ces eaux ammoniacales contiennent 13,7 % en masse de dioxyde de carbone, 9,7 % en masse d'ammoniac et de l'eau. L'hydantoïne formée est hydrolysée avec une solution de soude aqueuse à 50% en poids qui est introduite à un débit de 2310 kg/h ⑥. Le méthioninate de sodium est hydrolysé en méthionine par action de l'acide sulfurique à 98% qui est introduit en ⑦ à un débit de 2890 kg/h. On recueille en ⑧ une solution aqueuse contenant 15% de méthionine en poids avec un débit de 1986 kg/h. Le rendement de la méthionine par rapport à l'aldéhyde méthylthio propionique est de 96,0%.

## Revendications

1. Procédé de condensation d'un aldéhyde avec l'acide cyanhydrique en présence d'un tampon permettant de fixer le pH de la solution au dessus de 4,0 à l'exclusion des aminés **caractérisé en ce que** l'aldéhyde est un aldéhyde aliphatique contenant 1 à 6 atomes de carbone éventuellement substitués par un groupe alkyle, alkoxy ou alkylthio.

2. Procédé selon la revendication 1 **caractérisé en ce que** le pH de la solution est fixé entre 4,0 et 6,0 de préférence à 5.

3. Procédé selon la revendication 1 **caractérisé en ce que** le tampon est non volatil.

4. Procédé selon les revendications 1 à 3 **caractérisé en ce que** les tampons sont choisis parmi les tampons suivants :
- acide citrique et citrate de sodium ou acide citrique et hydroxyde de sodium
- acide phosphorique et phosphate acide de sodium ou acide phosphorique et hydroxyde de sodium,
- acide succinique et de succinate de sodium ou acide succinique et hydroxyde de sodium,
- acide acétique et acétate de sodium ou acide acétique et hydroxyde de sodium,
- hydrogénophtalate de potassium et hydroxyde de sodium.

5. Procédé selon la revendication 1 à 4 **caractérisé en ce que** l'aldéhyde est l'aldéhydè méthylthio propionique.

6. Procédé de condensation entre un aldéhyde et l'acide cyanhydrique en présence d'un tampon permettant de fixer le pH de la solution au dessus de 4,0 à l'exclusion des aminés **caractérisé en ce que** la condensation est une réaction d'absorption réactive entre l'aldéhyde liquide et l'acide cyanhydrique gazeux.

7. Procédé selon la revendication 6 **caractérisé en ce que** l'absorption réactive a lieu dans un contacteur gaz-liquide choisi parmi :
- une colonne à plateaux
- une colonne à garnissage
- une colonne à bulles
- un réacteur tubulaire
- une colonne à gouttes
- un réacteur à cuve agitée mécaniquement
- un réacteur à jet immergé.

8. Procédé selon la revendication 6 ou 7 **caractérisé en ce qu'**on ajoute en tête de la colonne d'absorption réactive une colonne de lavage.

9. Procédé de condensation entre un aldéhyde aliphatique et l'acide cyanhydrique en présence d'un tampon permettant de fixer le pH de la solution au dessus de 4,0 à l'exclusion des amines, ledit aldéhyde contenant 1 à 6 atomes de carbone, éventuellement substitués par un groupe alkyle, alkoxy ou alkylthio, **caractérisé en ce que** la condensation est une réaction entre l'aldéhyde liquide et une solution aqueuse d'acide cyanhydrique.

10. Procédé selon la revendication 9 **caractérisé en ce que** la réaction a lieu dans un réacteur piston adiabatique.

## Claims

1. Process for the condensation of an aldehyde with hydrogen cyanide in the presence of a buffer which makes it possible to set the pH of the solution above 4.0 with the exclusion of amines, **characterized in that** the aldehyde is an aliphatic aldehyde containing 1 to 6 carbon atoms optionally substituted with an alkyl, alkoxy or alkylthio group.

2. Process according to Claim 1, **characterized in that** the pH of the solution is set between 4.0 and 6.0, preferably at 5.

3. Process according to Claim 1, **characterized in that** the buffer is non-volatile.

4. Process according to Claims 1 to 3, **characterized in that** the buffers are chosen from the following buffers:
- citric acid and sodium citrate or citric acid and sodium hydroxide
- phosphoric acid and sodium hydrogen phosphate or phosphoric acid and sodium hydroxide,
- succinic acid and sodium succinate or succinic acid and sodium hydroxide,
- acetic acid and sodium acetate or acetic acid and sodium hydroxide,
- potassium hydrogen phthalate and sodium hydroxide.

5. Process according to Claims 1 to 4, **characterized in that** the aldehyde is methylthiopropionic aldehyde.

6. Process of condensation between an aldehyde and hydrogen cyanide in the presence of a buffer which makes it possible to set the pH of the solution above 4.0 with the exclusion of amines, **characterized in that** the condensation is a reactive absorption reaction between the liquid aldehyde and gaseous hydrogen cyanide.

7. Process according to Claim 6, **characterized in that** the reactive absorption takes place in a gas-liquid contact device chosen from:
- a column with plates
- a column with packing
- a bubble column
- a tubular reactor
- a drip column
- a reactor with a mechanically stirred tank
- a reactor with a submerged jet.

8. Process according to Claim 6 or 7, **characterized in that** a washing column is added to the head of the reactive absorption column.

9. Process of condensation between an aliphatic aldehyde and hydrogen cyanide in the presence of a buffer which makes it possible to set the pH of the solution above 4.0 with the exclusion of amines, the said aldehyde containing 1 to 6 carbon atoms, optionally substituted with an alkyl, alkoxy or alkylthio group, **characterized in that** the condensation is a reaction between the liquid aldehyde and an aqueous solution of hydrogen cyanide.

10. Process according to Claim 9, **characterized in that** the reaction takes place in an adiabatic piston reactor.

## Patentansprüche

1. Verfahren zur Kondensation eines Aldehyden mit Cyanwasserstoffsäure in Gegenwart eines Puffers, mit dem der pH-Wert der Lösung über 4,0 eingestellt werden kann, wobei Amine ausgenommen sind, **dadurch gekennzeichnet, daß** es sich bei dem Aldehyden um einen aliphatischen Aldehyden mit 1 bis 6 Kohlenstoffatomen handelt, der gegebenenfalls mit Alkyl, Alkoxy oder Alkylthio substituiert ist.

2. Verfahren nach Anspruch 1, **dadurch gekennzeichnet, daß** der pH-Wert der Lösung auf 4,0 bis 6,0 und vorzugsweise 5 eingestellt wird.

3. Verfahren nach Anspruch 1, **dadurch gekennzeichnet, daß** der Puffer nicht flüchtig ist.

4. Verfahren nach den Ansprüchen 1 bis 3, **dadurch gekennzeichnet, daß** die Puffer unter den folgenden Puffern ausgewählt sind:
- Citronensäure und Natriumcitrat oder Citronensäure und Natriumhydroxid;
- Phosphorsäure und Natriumhydrogenphosphat oder Phosphorsäure und Natriumhydroxid;
- Bernsteinsäure und Natriumsuccinat oder Bernsteinsäure und Natriumhydroxid;
- Essigsäure und Natriumacetat oder Essigsäure und Natriumhydroxid;
- Kaliumhydrogenphthalat und Natriumhydroxid.

5. Verfahren nach Anspruch 1 bis 4, **dadurch gekennzeichnet, daß** der Aldehyd der Methylthio-propionaldehyd ist.

6. Verfahren zur Kondensation eines Aldehyden mit Cyanwasserstoffsäure in Gegenwart eines Puffers, mit dem der pH-Wert der Lösung über 4,0 eingestellt werden kann, wobei Amine ausgenommen sind, **dadurch gekennzeichnet, daß** die Kondensation eine reaktive Absorption des flüssigen Aldehyden und der gasförmigen Cyanwasserstoffsäure ist.

7. Verfahren nach Anspruch 6, **dadurch gekennzeichnet, daß** die reaktive Absorption in einer Vorrichtung zum Inkontaktbringen eines Gases mit einer Flüssigkeit stattfindet, die ausgewählt ist unter:
- einer Bodenkolonne,
- einer Füllkörperkolonne,
- einem Blasensäulenreaktor,
- einem Röhrenreaktor,
- einer Tropfenkolonne,
- einem Reaktor mit mechanisch gerührtem Behälter, und
- einem Tauchstrahlreaktor.

8. Verfahren nach Anspruch 6 oder 7, **dadurch gekennzeichnet, daß** am Kopf der Kolonne für die reaktive Absorption eine Waschkolonne vorgesehen ist.

9. Verfahren zur Kondensation eines aliphatischen Aldehyden mit Cyanwasserstoffsäure in Gegenwart eines Puffers, mit dem der pH-Wert der Lösung über 4 eingestellt werden kann, wobei Amine ausgenommen sind und wobei der Aldehyd 1 bis 6 Kohlenstoffatome aufweist und gegebenenfalls mit Alkyl, Alkoxy oder Alkylthio substituiert ist, **dadurch gekennzeichnet, daß** die Kondensation eine Reaktion zwischen dem flüssigen Aldehyden und einer wäßrigen Cyanwasserstofflösung ist.

10. Verfahren nach Anspruch 9, **dadurch gekennzeichnet, daß** die Umsetzung in einem adiabatischen Kolbenreaktor stattfindet.
